(19) Europäisches Patentamt
European Patent Office
Office européen des brevets

(11) EP 0 725 066 B1

(12) **EUROPÄISCHE PATENTSCHRIFT**

(45) Veröffentlichungstag und Bekanntmachung des Hinweises auf die Patenterteilung:
**16.07.1997 Patentblatt 1997/29**

(51) Int. Cl.[6]: **C07D 307/28**, C07C 49/293, C07C 45/67

(21) Anmeldenummer: **95118935.6**

(22) Anmeldetag: **01.12.1995**

(54) **Verfahren zur Herstellung von Cyclopropylalkylketonen und 4,5-Dihydroalkylfuranen**

Process for the production of cyclopropylalkylketones and 4,5-dihydroalkylfuranes

Procédé pour la préparation de cyclopropylalkylkétones et 4,5-dihydroalkylfuranes

(84) Benannte Vertragsstaaten:
**CH DE FR GB LI**

(30) Priorität: **02.02.1995 DE 19503241**

(43) Veröffentlichungstag der Anmeldung:
**07.08.1996 Patentblatt 1996/32**

(73) Patentinhaber: **HÜLS AKTIENGESELLSCHAFT**
**45764 Marl (DE)**

(72) Erfinder:
- **Kaufhold, Manfred, Dr.**
**D-45770 Marl (DE)**
- **Feld, Marcel, Dr.**
**D-51147 Köln (DE)**

(56) Entgegenhaltungen:
**EP-A- 0 552 586** **EP-A- 0 610 819**

- **CHEMISTRY LETTERS, Bd. 11, 1975, Seiten 1149-1152, XP002003310 M.ASAOKA ET AL: "Alkylation reaction accompanied by dealkoxycarbonylation of beta-keto esters"**
- **TETRAHEDRON LETTERS, Nr. 49, 1975, Seiten 4389-4392, XP002003311 S.TAKEI ET AL: "A novel synthesis of cyclopropyl ketones via decarboxylative ring contractions"**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Rank Xerox (UK) Business Services
2.14.11/3.4

**Beschreibung**

Die Erfindung betrifft ein neues Verfahren zur gleichzeitigen Herstellung von Cyclopropylalkylketonen der Formel I und 4,5-Dihydroalkylfuranen der Formel II aus 3-Acyltetrahydrofuran-2-onen der Formel III nach dem Schema:

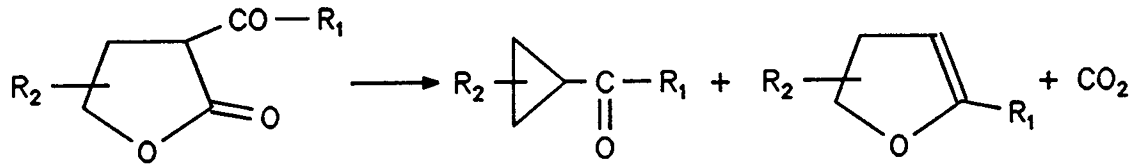

III I II

In diesem Reaktionsschema steht $R_1$ für Alkyl mit 1 bis 4 C-Atomen, Cyclohexyl oder Phenyl und $R_2$ für Wasserstoff, Alkyl mit 1 bis 4 C-Atomen oder Phenyl. Die Reaktion erfolgt dabei mit Hilfe eines Metallsalzes in einem hochsiedenden Lösemittel bei 160 bis 220 °C.

Verbindungen der Formeln I und II sind bekannt. Sie dienen als wichtige Ausgangsverbindungen zur Herstellung von Pflanzenschutzmitteln und Pharmaprodukten.

Die Herstellung von Cyclopropylalkylketonen aus 3-Acyltetrahydrofuran-2-onen durch Decarboxylierungsreaktionen ist literaturbekannt. So wird für die Reaktion nach Takei, Tetrahedron Letters No. 49, 4389-92 (1975), immer ein 10%iger molarer Überschuß an Alkalihalogenid, bezogen auf das Substrat, und außerdem ein polares Lösemittel, wie Dimethylsulfoxid oder Dimethylformamid, angewandt. Bei diesem Verfahren ist die Rückgewinnung der großen Alkalihalogenidmengen besonders aufwendig, da sowohl das Metallsalz als auch das Lösemittel sehr gut wasserlöslich sind.

Bei Asaoka, Chemistry Letters 11, 1149-52 (1975), wird $\alpha$-Acetyl-$\gamma$-butyrolacton mit unterschüssigem NaJ in Hexamethylphosphorsäuretriamid (HMPA), einem weiteren sehr polaren Lösemittel, umgesetzt. Nach der Destillation des Cyclopropylmethylketons erfordert die Rückgewinnung des Metallsalzes aus dem polaren Lösemittel auch hier ein aufwendiges Aufarbeitungsverfahren.

Nach EP-A-0 610 819 werden nur mit HMPA gute Ausbeuten erhalten, wobei das Lösemittel teuer und nicht unbedenklich sei. Im übrigen ist eine Gasphasenreaktion an einem Katalysatorbett Gegenstand dieser Anmeldung. Die Gasphasenreaktion erfordert jedoch eine spezielle Apparatur. Sie ist darüber hinaus mit einem großen technischen Aufwand verbunden, da der Katalysator auf einem festen Träger zuerst hergestellt und später regeneriert oder entsorgt werden muß.

In EP-A-0 552 586 wird ein großer Überschuß eines Halogenids in einem polaren Lösemittel, meist N-Methylpyrrolidon, vorgelegt, worauf bei der Reaktionstemperatur Acetylbutyrolacton zugetropft und Cyclopropylmethylketon destilliert wird. Wegen der großen Menge an Metallsalz und aus Umweltschutzgründen ist bei diesem Verfahren eine Rückgewinnung des Metallsalzes unbedingt erforderlich. Die Rückgewinnung ist dabei auch hier sehr aufwendig.

Aufgabe der vorliegenden Erfindung war es daher, ein einfaches Verfahren bereitzustellen, das in üblichen Rührapparaturen durchführbar ist, bei dem der Katalysator auf einfache Weise zurückgewonnen werden kann und bei dem nur wenig hochsiedende Abfallprodukte entstehen.

Die Aufgabe wird erfindungsgemäß dadurch gelöst, daß man für die Reaktion als Lösemittel ein N-Alkyllactam der Formel IV

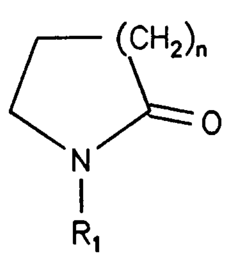

IV ,

wobei $R_1$ ein Alkyl mit 4 bis 12 C-Atomen, Cycloalkyl, Benzyl oder ein substituiertes Benzyl gemäß

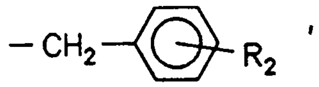

worin $R_2$ für Alkyl mit 1 bis 4 C-Atomen steht, und n eine Zahl von 1 bis 6 sein kann, oder ein N-Acylmorpholin der Formel V

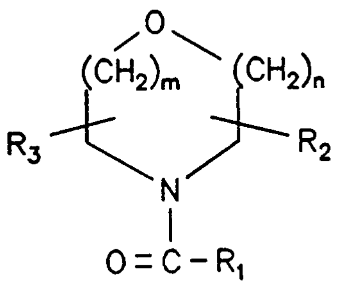

V ,

wobei $R_1$ ein Alkylrest mit 4 bis 12 C-Atomen, Cycloalkyl, Benzyl oder ein substituiertes Benzyl gemäß

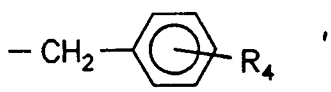

worin $R_4$ für Alkyl mit 1 bis 4 C-Atomen steht, $R_2$ und $R_3$ unabhängig voneinander Wasserstoff oder Alkyl mit 1 bis 4 C-Atomen sein kann und n und m zusammen eine Zahl von 1 bis 10 ergeben können, verwendet. Außerdem ist die Reaktion in der Weise durchzuführen, daß man das Metallsalz und III im molaren Überschuß zum Metallsalz im Lösemittel vorlegt, auf 160 bis 220 °C erwärmt, dann weiteres III zugibt und dabei I und II abdestilliert.

Beispiele für Cyclopropylalkylketone I sind Cyclopropylmethylketon, Cyclopropylethylketon, Cyclopropylphenylketon, Cyclopropylcyclohexylketon und 1-Methyl-cyclopropylphenylketon.

Beispiele für 4,5-Dihydroalkylfurane II sind 4,5-Dihydro-2-methylfuran, 4,5-Dihydro-2,5-dimethylfuran, 4,5-Dihydro-2-phenylfuran, 4,5-Dihydro-2-cyclohexylfuran und 4,5-Dihydro-5-methyl-2-phenylfuran.

Geeignete Ausgangsverbindungen III sind beispielsweise $\alpha$-Acetyl-$\gamma$-butyrolacton, 3-Acetyl-5-methyltetradydrofuran-2-on, $\alpha$-Benzoyl-$\gamma$-butyrolacton, $\alpha$-Acetyl-$\alpha$-phenyl-$\gamma$-butyrolacton und $\alpha$-Benzoyl-$\alpha$-methyl-$\gamma$-butyrolacton.

Als Metallsalze kommen vor allem Alkali- und Erdalkalihalogenide in Betracht. Dabei werden Alkalihalogenide vorzugsweise eingesetzt. Beispiele dafür sind LiBr, LiJ, KCl, NaBr, KBr, NaJ und KJ, wobei NaJ und KJ ganz besonders bevorzugt werden.

Die Lösemittel IV weisen vorzugsweise 8 bis 16 C-Atome auf. Beispiele dafür sind 1-(n-Butyl)pyrrolidon-2, 1-Cyclohexylpyrrolidon-2, 1-Benzylpyrrolidon-2 und 1-(n-Octyl)caprolactam.

Die Lösemittel V enthalten im allgemeinen 8 bis 20 C-Atome. Beispiele dafür sind 4-Benzoyl-morpholin, 4-Hexanoyl-morpholin, 4-Lauroyl-morpholin und 4-Benzoyl-2,6-dimethylmorpholin.

In dem verwendeten Lösemittel werden Metallsalz und III vorzugsweise im molaren Verhältnis 1 : 2 bis 1 : 10 und im besonderen im molaren Verhältnis von 1 : 2,5 bis 1 : 5 vorgelegt.

Bei den Lösemitteln IV und V handelt es sich um hochsiedende, polare und dabei wenig wasserlösliche Verbindungen. Wegen ihrer geringen Wasserlöslichkeit ermöglichen sie es, daß die Metallsalze nach der Destillation von I und II und Abkühlung des verbleibenden Sumpfes durch Wasserwäsche in einfacher Weise abgetrennt werden können.

Die Lösemittel können in reiner Form oder als Gemische eingesetzt werden. Sie können auch bis zu 50 %, bezogen auf die Gesamtmenge an Lösemittel, durch hochsiedende, polare und wasserlösliche Lösemittel, wie Dimethylformamid, Dimethylsulfoxid oder N-Methylpyrrolidon, substituiert werden. Anders als bei Verwendung von nur wasserlöslichen Lösemitteln gelingt es so immer noch, Hochsieder wirkungsvoll abzutrennen und die Metallsalze durch Wasserwäsche in einfacher Weise zurückzugewinnen.

Wesentlich für das erfindungsgemäße Verfahren ist, daß III im molaren Überschuß zum Metallsalz vorgelegt wird. Das ergeben die experimentellen Daten. Bei diesem Überschuß können dann die Produkte I und II mit Hilfe der speziellen Lösemittel überraschend einfach und wirtschaftlich hergestellt werden.

Im Gegensatz zu der in EP-A-0 610 819 geäußerten Auffassung werden auch mit den weniger polaren und weniger wasserlöslichen erfindungsgemäßen Lösemitteln hohe Ausbeuten erzielt. Anders als im Stand der Technik entstehen hochsiedende Produkte nur in geringen Mengen. Statt dessen fällt II als weiteres leicht siedendes Produkt an. Die geringen Mengen an Hochsiedern haben den wirtschaftlichen Vorteil, daß der Sumpf seltener aufgearbeitet werden muß und daß daher die Standzeit bis zum Abbruch der Reaktion erhöht wird.

Es wird also weniger Salz eingesetzt, das seltener und dann in einfacher Weise zurückgewonnen wird. Gleichzeitig gewinnt man mit II ein weiteres wertvolles Zwischenprodukt.

Zur Durchführung des erfindungsgemäßen Verfahrens legt man das Lösemittel oder ein Lösemittelgemisch und III vor und gibt dann das Metallsalz hinzu. Die Konzentration des Metallsalzes beträgt meist 5 bis 20 %, bezogen auf das Gemisch aus Lösemittel und III. Im besonderen wird ein Molverhältnis von Metallsalz und III von 1 : 3 eingestellt. Man erwärmt auf 160 bis 220 °C, vorzugsweise auf 180 bis 200 °C und dosiert weiteres III zu. Das dann sich bildende I und II wird abdestilliert.

Die folgenden Beispiele sollen die Erfindung verdeutlichen.

Vergleichsbeispiel A

In einer Glasapparatur aus einem Dreihalskolben mit Rührer, Thermometer, Destillationskolonne, Destillationsbrücke und Vorlage werden eingesetzt:

| | |
|---|---|
| 100 g | 1-(n-Octyl)caprolactam (NOC) |
| 40 g (0,31 Mol) | 3-Acetylbutyrolacton (ABL) |
| 15 g (0,1 Mol) | NaJ, getrocknet |

Die Produkte werden vermischt und auf 180 °C erhitzt. Dabei fällt kein Destillat an.

Beispiel 1

Man verwendet die Apparatur von Vergleichsbeispiel A und legt die dort genannten Verbindungen in den angegebenen Mengen vor. Man erhitzt auf 180 °C und dosiert dann weiteres ABL in einer Menge von 40 bis 45 g/h zu. Nachdem 5 g ABL zugegeben sind, steigt die Kopftemperatur der Kolonne auf 105 °C an. Nach weiteren 15 g ABL fallen 12 g Destillat an. Danach stellt sich eine Destillatmenge von 36 bis 40 ml/h ein.

Es werden 1 241 g ABL auf diese Weise zugegeben. Die Gesamtmenge einschließlich der vorgelegten 40 g beträgt dann 1 281 g ABL (10 Mol). Dabei fallen 787 g Destillat und 163 g Sumpfprodukt an. Demnach bilden sich, wenn man OCL und NaJ abzieht, nur 48 g Hochsieder. Das sind 3,7 %, bezogen auf den Einsatz.

Das Destillat besteht aus

| | |
|---|---|
| 674 g | Cyclopropylmethylketon (CPMK) und |
| 107 g | 4,5-Dihydro-2-methylfuran (DHMF). |

Die Ausbeute an CPMK beträgt 80,1 % und die an DHMF 12,7 %. Die Gesamtausbeute liegt demnach bei ca. 93 %.

Zum Sumpfprodukt werden unter Rühren 100 g Wasser gegeben. Nach Abstellen des Rührers bilden sich sogleich zwei klar abgegrenzte, leicht trennbare Phasen. Der Waschvorgang wird mit 30 g Wasser wiederholt. Hochsieder und NaJ sind dann voneinander getrennt.

Beispiel 2

In der oben beschriebenen Apparatur werden vorgelegt:

| | |
|---|---|
| 100 g | NOC |
| 100 g | N-Methylpyrrolidon (NMP) |
| 77 g (0,6 Mol) | ABL, Reinheit 98,5 % |
| 30 g (0,2 Mol) | NaJ, getrocknet |

Unter Rühren erwärmt man auf 180 °C und dosiert dann 90 ml/h ABL zu, wobei 65 bis 75 ml/h Leichtsieder abdestillieren. Insgesamt werden 9 810 g ABL zudosiert. Die Gesamtmenge liegt bei 9 887 g ABL (98,5 %).

Es fallen insgesamt

| | |
|---|---|
| 5 355 g (63,7 Mol) | CPMK, Ausbeute 83 %, und |
| 578 g ( 6,87 Mol) | DHMF, Ausbeute 9,0 %, |

an. Das entspricht einer Gesamtausbeute an Wertprodukt von 92 %.

Die Laufzeit beträgt über 100 h. In dieser Zeit nimmt der Rückstand nur um 340 g oder 3,4 %, bezogen auf den Einsatz, zu.

Der Rückstand wird durch Andestillieren von Leichtsiedern befreit und dann zur Rückgewinnung des Natriumjodids 3 mal mit je 100 ml Wasser gewaschen. Insgesamt werden so 28,8 g NaJ als wäßrige Lösung erhalten. Das sind 96 % des Einsatzes.

Das Wasser wird dann abdestilliert. Dabei fällt ein homogener flüssiger Rückstand an, der das NaJ in gelöster Form sowie 89 g NMP und 7 g NOC enthält. Diese leicht handhabbare Katalysatorlösung wird in die Synthese wieder eingesetzt.

Beispiele 3 bis 6

Man verfährt wie in Beispiel 1, ersetzt jedoch NOC durch Lösemittelgemische oder andere Lösemittel. Die Ergebnisse gehen aus Tabelle 1 hervor.

Tabelle 1

| Beispiel | Lösemittel | Ausbeute | | |
|---|---|---|---|---|
| | | CPMK | DHMF | gesamt |
| 3 | 90 g NOC + 10 g NMP | 82,2 | 10,2 | 92,4 |
| 4 | 70 g NOC + 30 g NMP | 85,0 | 8,2 | 93,2 |
| 5 | 100 g 1-Cyclohexylpyrrolidon-2 | 87,1 | 6,5 | 93,6 |
| 6 | 100 g 1-Benzylpyrrolidon-2 | 83,8 | 9,5 | 93,3 |

Beispiel 7

Man verwendet eine Apparatur, die aus einer 35-l-Rührapparatur, einer Destillationskolonne mit Destillationsapparatur, Kühler, Vorlagen und den dazugehörigen Pumpen besteht, und setzt ein:

| | |
|---|---|
| 6,0 kg | NOC |
| 6,0 kg | NMP |
| 4,5 kg | ABL |
| 1,8 kg | NaJ |

Die Durchführung erfolgt wie in Beispiel 2. Dabei werden in 100 h 236 kg ABL kontinuierlich zugepumpt. Danach

beträgt die Zunahme an hochsiedendem Rückstand nur 2,0 kg oder 0,85 %. Diese geringe Zunahme an Hochsiedern zeigt den Vorteil des erfindungsgemäßen Verfahrens besonders deutlich.

Ausbeuten (verlustfrei gerechnet):

| | |
|---|---|
| 92,0 % | CPMK |
| 5,3 % | DHMF |
| 97,3 % | Gesamtausbeute an Wertprodukt |

**Patentansprüche**

1. Verfahren zur gleichzeitigen Herstellung von Cyclopropylalkylketonen der Formel I und Alkyl-4,5-dihydro-2-alkylfuranen der Formel II aus 3-Acyltetrahydrofuran-2-onen der Formel III

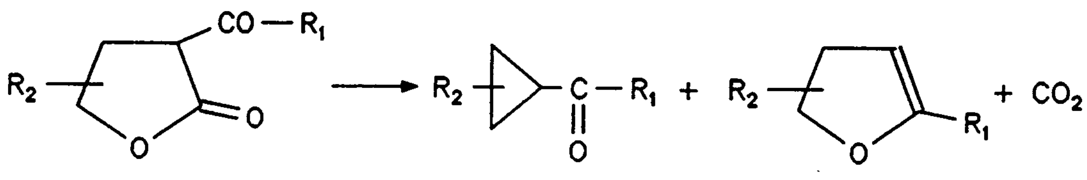

III I II

wobei

$R_1$ = Alkyl mit 1 bis 4 C-Atomen, Cyclohexyl oder Phenyl und
$R_2$ = H, Alkyl mit 1 bis 4 C-Atomen oder Phenyl ist,

durch Umsetzung mit einem Metallsalz in einem hochsiedenen Lösemittel bei 160 bis 220 °C, dadurch gekennzeichnet, daß man als Lösemittel ein N-Alkyllactam der Formel IV

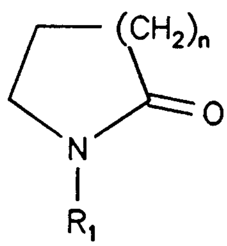

IV ,

wobei

$R_1$ = Alkyl mit 4 bis 12 C-Atomen, Cycloalkyl, Benzyl oder substituiertes Benzyl gemäß

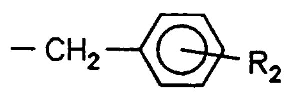

,

$R_2$ = Alkyl mit 1 bis 4 C-Atomen und
n = 1 bis 6 ist,

oder ein N-Acylmorpholin der Formal V

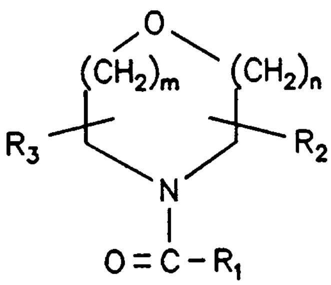

V ,

wobei

$R_1$ = Alkyl mit 4 bis 12 C-Atomen, Cycloalkyl, Benzyl oder substituiertes Benzyl gemäß

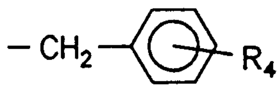

,

$R_4$ = Alkyl mit 1 bis 4 C-Atomen,
$R_2$, $R_3$ = H oder Alkyl mit 1 bis 4 C-Atomen und n + m = 1 bis 10 ist,

verwendet und für die Reaktion das Metallsalz und III im molaren Überschuß zum Metallsalz im Lösemittel vorlegt, auf 160 bis 220 °C erwärmt, dann weiteres III zugibt und dabei I und II abdestilliert.

**2.** Verfahren nach Anspruch 1,
dadurch gekennzeichnet,
daß als Metallsalz ein Alkalihalogenid eingesetzt wird.

**3.** Verfahren nach Anspruch 2,
dadurch gekennzeichnet,
daß NaJ oder KJ verwendet wird.

**4.** Verfahren nach Anspruch 1,
dadurch gekennzeichnet,
daß Metallsalz und III im molaren Verhältnis von 1 : 2 bis 1 : 10, vorzugsweise von 1 : 2,5 bis 1 : 5 vorgelegt werden.

**5.** Verfahren nach Anspruch 1,
dadurch gekennzeichnet,
daß das Metallsalz nach der Destillation von I und II durch Wasserwäsche zurückgewonnen wird.

**6.** Verfahren nach Anspruch 1,
dadurch gekennzeichnet,
daß neben den Lösemitteln IV und V hochsiedende, polare und wasserlösliche Lösemittel in Mengen bis zu 50 %, bezogen auf die gesamte Lösemittelmenge, eingesetzt werden.

**Claims**

**1.** A process for the simultaneous preparation of cyclopropyl alkyl ketones of the formula I and alkyl-4,5-dihydro-2-alkylfurans of the formula II from 3-acyltetrahydrofuran-2-ones of the formula III

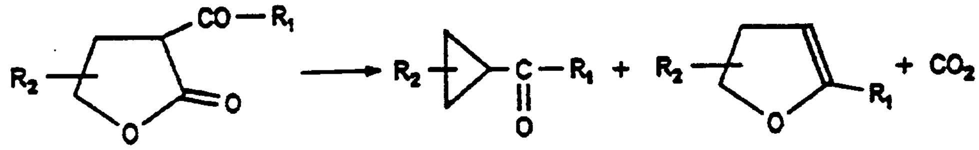

III I II

where

$R_1$ = alkyl having 1 to 4 carbon atoms, cyclohexyl or phenyl and
$R_2$ = H, alkyl having 1 to 4 carbon atoms or phenyl,

by reaction with a metal salt in a high-boiling solvent at 160 to 220°C, characterized in that an N-alkyllactam of the formula IV

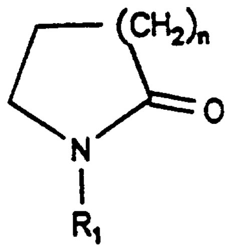

IV ,

where

$R_1$ = alkyl having 4 to 12 carbon atoms, cycloalkyl, benzyl or substituted benzyl according to

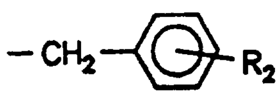

,

$R_2$ = alkyl having 1 to 4 carbon atoms and
n = 1 to 6,

or an N-acylmorpholine of the formula V

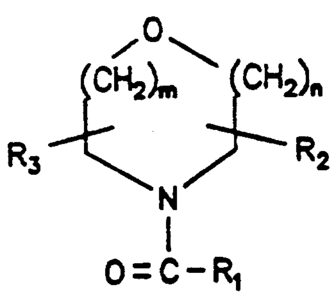

V ,

where

$R_1$ = alkyl having 4 to 12 carbon atoms, cycloalkyl, benzyl or substituted benzyl according to

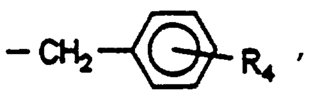

$R_4$ = alkyl having 1 to 4 carbon atoms,
$R_2$, $R_3$ = H or alkyl having 1 to 4 carbon atoms and $n + m = 1$ to 10 ,

is used as solvent and, for the reaction, the metal salt and III in a molar excess with respect to the metal salt are placed in the solvent, heated to 160 to 220°C, then further III is added and I and II are distilled off in the course of this.

**2.** A process according to claim 1, characterized in that an alkali metal halide is used as metal salt.

**3.** A process according to claim 2, characterized in that NaI or KI is used.

**4.** A process according to claim 1, characterized in that metal salt and III are introduced in a molar ratio of 1 : 2 to 1 : 10, preferably of 1 : 2.5 to 1 : 5.

**5.** A process according to claim 1, characterized in that the metal salt, after the distillation of I and II, is recovered by washing with water.

**6.** A process according to claim 1, characterized in that, esides the solvents IV and V, high-boiling, polar and water-soluble solvents are used in amounts up to 50%, based on the total amount of solvent.

**Revendications**

**1.** Procédé de fabrication simultanée de cyclopropylalkylcétones de la formule I et d'alkyl-4,5-dihydro-alkylfuranes de la formule II à partir de 3-acyltétrahydrofuranes-2-ones de la formule III.

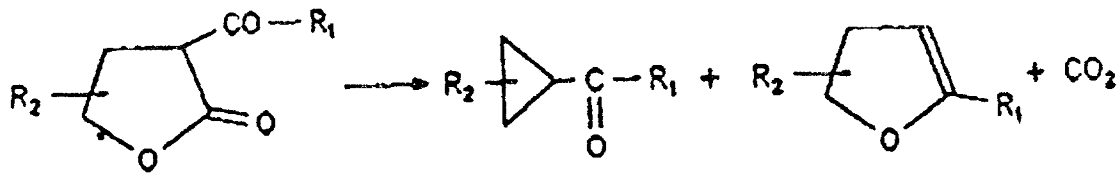

III I II

dans laquelle :

$R_1$ = alkyle avec 1 à 4 atomes de carbone, cyclohéxyle ou phényle et,
$R_2$ = H, alkyle avec 1 à 4 atomes de carbone ou phényle,

par réaction avec un sel métallique dans un solvant à point d'ébullition élevé à 160 jusqu'à 220°C, caractérisé en ce qu'
on utilise comme solvant un N-alkylactame de la formule IV,

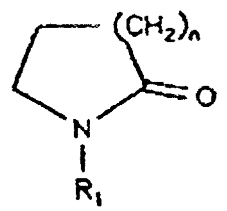

IV

dans laquelle :

$R_1$ = alkyle avec 4 à 12 atomes de carbone, cycloalkyle, benzyle ou benzyle substitué selon :

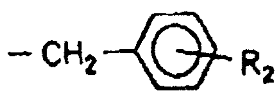

$R_2$ = alkyle avec 1 à 4 atomes de carbone et,
n = 1 à 6, ou une N-acylmorpholine de la formule V :

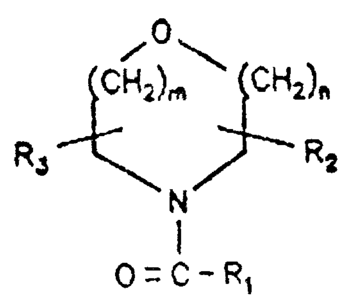

V

dans laquelle :

$R_1$ = alkyle avec 4 à 12 atomes de C, cycloalkyle, benzyle ou benzyle substitué selon

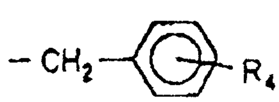

$R_4$ = alkyle avec 1 à 4 atomes de C,
$R_2$, $R_3$ = H ou alkyle avec 1 à 4 atomes de C et n + m = 1 à 10 ,

et pour la réaction on dispose le sel métallique et III en excès molaire par rapport au sel métallique dans le solvant, on chauffe à 160 jusqu'à 220°C, ensuite on ajoute à nouveau du III et en outre on élimine I et II par distillation.

**2.** Procédé selon la revendication 1,
caraccérisé en ce que
comme sel métallique on utilise un halogénure alcalin.

**3.** Procédé selon la revendication 2,
caractérisé en ce qu'

on utilise NaJ ou KJ.

**4.** Procédé selon la revendication 1,
caractérisé en ce que
le sel métallique et III sont prédisposés dans le rapport de 1:2 à 1:10, de préférence de 1:2,5 à 1:5.

**5.** Procédé selon la revendication 1,
caractérisé en ce que
le sel métallique après la distillation de I et II est récupéré par des lavages à l'eau.

**6.** Procédé selon la revendication 1,
caractérisé en ce qu'
à côté des solvants IV et V sont mis en oeuvre des solvante à point élevé d'ébullition, polaires et solubles dans l'eau en quantités allant jusqu'à 50 %, par rapport à la quantité totale de solvants.